# EUROPEAN PATENT SPECIFICATION

(11) **EP 3 082 721 B1**
(45) Date of publication and mention of the grant of the patent: **17.04.2019**
(21) Application number: 13817828.0
(22) Date of filing: 19.12.2013
(51) Int. Cl.: A61K 8/27, A61K 8/44, A61Q 11/00

(54) **ANTI-MALODOR ORAL CARE COMPOSITION**
MUNDPFLEGEZUSAMMENSETZUNG GEGEN SCHLECHTE GERÜCHE
COMPOSITION DE SOINS BUCCAUX CONTRE LA MAUVAISE HALEINE

(43) Date of publication of application: 26.10.2016
(73) Proprietor: Colgate-Palmolive Company, New York, NY 10022 (US)
(72) Inventor: VAZQUEZ, Joe, Hamilton Township, NJ 08690 (US); TRIVEDI, Harsh M., Hillsborough, NJ 08844 (US); KILPATRICK-LIVERMAN, Latonya, Princeton, NJ 08542 (US); LAVENDER, Stacey, Chesterfield, NJ 08515 (US)
(74) Representative: Wibbelmann, Jobst
(86) International application number: PCT/US2013/076354
(87) International publication number: WO 2015/094254

(56) References cited:
- WO-A1-2011/123123
- WO-A1-2011/162756
- WO-A1-2014/088572
- WO-A1-2014/088573
- WO-A1-2014/088575
- WO-A1-2014/098825
- GB-A- 2 481 630
- US-A- 5 370 865

## Description

### BACKGROUND

One of the major contributors to malodor in the oral cavity is the bacteria present on the soft and hard oral tissues. Manual brushing and rinsing help to remove the bacteria, but they eventually repopulate over a period of time.

Zinc ions act as a bacteriostatic agent by binding with bacteria and slowing down the growth phase, leading to slower cell division and repopulation. Zinc salts in oral care products are known to react with sulfur compounds found in bad breath. For example, the reaction of zinc oxide with hydrogen sulfide produces odorless zinc sulfide.

However, there is still a need in the art for oral care compositions which have even greater efficacy in reducing oral malodor than previous zinc ion-containing compositions.

### BRIEF SUMMARY

A first aspect of the present invention provides an oral care composition comprising: arginine in free or salt form; and zinc ions, wherein the zinc ions are present in the composition at a concentration of from 0.0005 mols/100g to 0.00062 mols/100g, based on the total weight of the composition

WO-A-2011/162756 and US-A-5 370 865 disclose compositions comprising arginine and zinc oxide.

Optionally, the zinc ions are present in the composition at a concentration of from 0.00052 mols/100g to 0.00062 mols/100g, based on the total weight of the composition.

Optionally, the arginine is present in the composition at a concentration of from 0.1 weight % to 1 weight %, based on the total weight of the composition.

Optionally, the arginine is present in the composition at a concentration of from 0.5 weight % to 0.8 weight %, based on the total weight of the composition.

Optionally, the zinc ions are provided, but not limited to by zinc oxide, zinc lactate, zinc chloride, zinc citrate, zinc acetate, zinc borate, zinc butyrate, zinc carbonate, zinc formate, zinc gluconate, zinc glycerate, zinc glycolate, zinc phosphate, zinc picolinate, zinc proprionate, zinc salicylate, zinc silicate, zinc stearate, zinc tartrate, zinc undecylenate, zinc ricinoleate, zinc nitrate, zinc sulfate or mixtures thereof.

Optionally, the zinc ions are provided by zinc oxide.

Optionally, the zinc ions are provided by zinc lactate.

Optionally, the zinc ions are provided by zinc chloride.

Optionally, the zinc ions are provided by zinc citrate.

Optionally, the arginine is present as free arginine.

Optionally, the arginine is present as arginine bicarbonate.

Optionally, the arginine is present as a peptide having from 2-4 amino acid units at least one of which is arginine.

Optionally, the composition further comprises one or more agents selected from abrasives, diluents, additional bicarbonate salts, pH modifying agents, surfactants, foam modulators, thickening agents, humectants, sweeteners, flavorants, pigments, antibacterial agents, anticaries agents, anticalculus or tartar control agents, whitening agents, and mixtures thereof.

Optionally, the composition is a toothpaste, a gel, a mouthwash, a mouthrinse, a lozenge, a spray, a gum, a tablet, or a film.

Optionally, the composition is a mouthwash.

Optionally, the composition is a toothpaste.

In a second aspect, the present invention provides said oral care composition for use in a method of reducing oral malodor in an oral cavity of a subject, the method comprising applying said oral care composition of the present invention to the oral cavity.

### DETAILED DESCRIPTION

The following description of the preferred embodiments is merely exemplary in nature and is in no way intended to limit the invention, its application, or uses.

As used throughout, ranges are used as shorthand for describing each and every value that is within the range. Any value within the range can be selected as the terminus of the range.

As referred to herein, all compositional percentages are by weight of the total composition unless otherwise indicated. As referred to herein, "ppm" (parts per million) refers to ppm by weight, unless otherwise indicated. As referred to herein, all ratios refer to weight ratios, unless otherwise indicated.

The present inventors have found that oral care compositions comprising a combination of zinc ions and arginine as defined in the claims show a surprisingly high efficacy in reducing levels of
volatile sulfur compounds (VSCs) as compared to compositions containing either zinc ions alone or arginine alone.

The present invention provides an oral care composition comprising: arginine in free or salt form; and zinc ions (Zn²° ions), wherein the zinc ions are present in the composition at a concentration of from 0.0005 mols/100g to 0.00062 mols/100g, based on the total weight of the composition.

In some embodiments, the zinc ions are present in the composition at a concentration
from 0.00052 mols/100g to 0.00062 mols/100g, based on the total weight of the composition.

In some embodiments, the zinc ions are provided by a zinc compound which includes, but is not limited to zinc oxide, zinc lactate, zinc chloride, zinc citrate, zinc acetate, zinc borate, zinc butyrate, zinc carbonate, zinc formate, zinc gluconate, zinc glycerate, zinc glycolate, zinc phosphate, zinc picolinate, zinc proprionate, zinc salicylate, zinc silicate, zinc stearate, zinc tartrate, zinc undecylenate, zinc phosphate, zinc ricinoleate, zinc nitrate, zinc sulfate or mixtures thereof.

In some embodiments, the zinc ions are provided by zinc oxide, zinc lactate, zinc chloride, zinc citrate, or mixtures thereof.

In some embodiments, the zinc ions are provided by zinc oxide. In some embodiments, the zinc ions are provided by zinc lactate. In some embodiments, the zinc ions are provided by zinc chloride. In some embodiments, the zinc ions are provided by zinc citrate.

In some embodiments, the arginine is present in the composition at a concentration of from 0.1 weight % to 1 weight %, or from 0.5 weight % to 0.8 weight %, based on the total weight of the composition.

In some embodiments, the arginine is present as free arginine. In some embodiments, the arginine is present as a salt.

In some embodiments, when the arginine is in salt form, if may be a salt which includes, but is not limited to, arginine bicarbonate, arginine hydroxide, arginine carbonate, arginine phosphate and mixtures thereof.

Because of the surprising nature of the ability of the combination of arginine in free or salt form with a zinc ion to reduce volatile sulfur compounds (VSCs), another embodiment of the invention is oral care compositions consisting essentially of a combination of zinc ions and arginine.

The composition can also contain an orally acceptable carrier. Any conventional carrier that is typically used to make an oral care composition can be used. In some embodiments, the oral care composition is a toothpaste, a gel, a mouthwash, a mouthrinse, a lozenge (which may be dissolvable or chewable), a spray, a gum, a tablet, or a film (which may be wholly or partially dissolvable, or indissolvable).

In some embodiments, the oral care composition is a mouthwash. In some embodiments, the oral care composition is a toothpaste.

In some embodiments, the oral care composition further may comprise one or more agents selected from abrasives, diluents, additional bicarbonate salts, pH modifying agents, surfactants, foam modulators, thickening agents, humectants, sweeteners, flavorants, pigments, antibacterial agents, anticaries agents, anticalculus or tartar control agents, and mixtures thereof.

In some embodiments, particularly (but not limited to) those embodiments, wherein the oral care composition is a toothpaste, the compositions of the present invention may further comprise an abrasive.

Abrasives that may be used include silica abrasives such as precipitated or hydrated silicas having a mean particle size of up to about 20 microns, such as Zeodent 105 and Zeodent 114 marketed by J.M. Huber Chemicals Division, Havre de Grace, Md. 21078, or Sylodent 783 marketed by Davison Chemical Division of W.R. Grace & Company. Abrasives such as Sorbosil AC 43 from PQ Corporation may also be included. Other useful dentifrice abrasives include aluminium oxide, aluminum silicate, calcined alumina, bentonite or other siliceous materials, insoluble phosphates, and mixtures thereof.

The abrasive may be present in an amount of from 5 to 30 weight % based on the weight of the composition, optionally from 10 to 20 weight % based on the weight of the composition.

In certain embodiments, particularly (but not limited to) those embodiments wherein the oral care composition is a mouthwash or mouthrinse, the compositions may be free of abrasives.

In some embodiments, the oral care compositions of the present invention may comprise at least one additional bicarbonate salt (i.e. in addition to any arginine bicarbonate which may be present in certain embodiments), useful for example to impart a "clean feel" to teeth and gums due to effervescence and release of carbon dioxide. Any orally acceptable bicarbonate can be used, including without limitation, alkali metal bicarbonates such as sodium and potassium bicarbonates, ammonium bicarbonate and the like. The one or more additional bicarbonate salts are optionally present in a total amount of about 0.1 wt, % to about 50 wt. %, for example about 1 wt. % to 20 wt. %, by total weight of the composition.

In some embodiments, the compositions of the present invention may comprise at least one pH modifying agent. Such agents include acidifying agents to lower pH, basifying agents to raise pH, and buffering agents to control pH within, a desired range. For example, one or more compounds selected from acidifying, basifying and buffering agents can be included to provide a pH of 2 to 10, or in various illustrative embodiments, 2 to 8, 3 to 9, 4 to 8, 5 to 7, 6 to 10, 7 to 9, etc. Any orally acceptable pH modifying agent can be used, including without limitation, carboxylic, phosphoric and sulfonic acids, acid salts (*e.g*., monosodium citrate, disodium citrate, monosodium malate, *etc*.), alkali metal hydroxides such as sodium hydroxide, carbonates such as sodium carbonate, bicarbonates (in addition to any arginine bicarbonate which might be present in certain embodiments), sesquicarbonates, borates, silicates, phosphates (*e.g*., monosodium phosphate, trisodium phosphate), imidazole and the like. One or more pH modifying agents are optionally present in a total amount effective to maintain the composition in an orally acceptable pH range.

In a still further embodiment, the compositions of the invention, may comprise at least one surfactant. Any orally acceptable surfactant, most of which are anionic, nonionic or amphoteric, can be used. Suitable anionic surfactants include without limitation, watersoluble salts of C₈₋₂₀ alkyl sulfates, sulfonated monoglycerides of C₈₋₂₀ fatty acids, sarcosinates, taurates and the like. Illustrative examples of these and other classes include sodium lauryl sulfate, sodium coconut monoglyceride sulfonate, sodium lauryl sarcosinate, sodium lauryl isoethionate, sodium laureth carboxylate and sodium dodecyl benzenesulfonate. Suitable nonionic surfactants include without limitation, poloxamers, polyoxyethylene sorbitan esters, fatty alcohol ethoxylates, alkylphenol ethoxylates, tertiary amine oxides, tertiary phosphine oxides, dialkyl sulfoxides and the like. Suitable amphoteric surfactants include without limitation, derivatives of C₈₋₂₀ aliphatic secondary and tertiary amines having an anionic group such as carboxylate, sulfate, sulfonate, phosphate or phosphonate. Betaines may also be used, a suitable example of which is cocoamidopropyl betaine. One or more surfactants are optionally present in a total amount of about 0.01 weight % to about 10 wt. %, for example, from about 0.05 wt. % to about 5 wt. %, or from about 0.1 wt. % to about 2 wt. % by total weight of the composition.

In some embodiments, the compositions of the invention may comprise at least one foam modulator, useful for example to increase amount, thickness or stability of foam generated by the composition upon agitation. Any orally acceptable foam modulator can be used, including without limitation, polyethylene glycols (PEGs), also known as polyoxyethylenes. High molecular weight PEGs are suitable, including those having an average molecular weight of 200,000 to 7,000,000, for example 500,000 to 5,000,000, or 1,000,000 to 2,500,000. One or more PEGs are optionally present in a total amount of about 0.1 wt. % to about 10 wt. %, for example from about 0.2 wt. % to about 5 wt. %, or from about 0,25 wt. % to about 2 weight %, by total weight of the composition.

In certain embodiments, particularly (but not limited to) those embodiments wherein the oral care composition is a mouthwash or mouthrinse, the compositions may be free of foam modulating agents.

In some embodiments, the compositions of the present invention may comprise at least one thickening agent, useful for example to impart a desired consistency and/or mouth feel to the composition. Any orally acceptable thickening agent can be used, including without limitation, carbomers, also known as carboxyvinyl polymers, carrageenans, also known as Irish moss and more particularly 1-carrageenan (iota-carrageenan), cellulosie polymers such as hydroxyethylcellulose, carboxymethylcellulose (CMC) and salts thereof, *e.g.,* CMC sodium, natural gums such as karaya, xanthan, gum arabie and tragacanth, colloidal magnesium aluminum silicate, colloidal silica and the like. A preferred class of thickening or gelling agents includes a class of homopolymers of acrylic acid crosslinked with an alkyl ether of pentaerythritol or an alkyl ether of sucrose, or carbomers. Carbomers are commercially available from B. F. Goodrich as the Carbopol® series. Particularly preferred Carbopols include Carbopol 934, 940, 941, 956, 974P, and mixtures thereof. Silica thickeners such as Hi-Sil®DT 267 (from PPG Industries) may also be used. One or more thickening agents are optionally present in a total amount of from about 0.01 wt. % to 15 weight %, for example from about 0.1 weight % to about 10 weight %, or from about 0.2 wt. % to about 5 weight %, by total weight of the composition.

In some embodiments, the compositions of the invention may comprise at least one viscosity modifier, useful for example to help inhibit settling or separation of ingredients or to promote re-dispersibility upon agitation of a liquid composition. Any orally acceptable viscosity modifier can be used, including without limitation, mineral oil, petrolatum, clays and organomodified clays, silica and the like. One or more viscosity modifiers are optionally present in a total amount of from about 0.01 wt. % to about 10 wt. %, for example, from about 0.1 weight % to about 5 weight %, by total weight of the composition.

In some embodiments, the compositions of the invention may comprise at least one humectant. Any orally acceptable humectant can be used, including without limitation, polyhydric alcohols such as glycerin, sorbitol (optionally as a 70 weight % solution in water), xylitol or low molecular weight polyethylene glycols (PEGs). Most humectants also function as sweeteners. One or more humectants are optionally present in a total amount of from about 1 weight % to about 70 weight %, for example, from about 1 weight % to about 50 weight %, from about 2 weight % to about 25 weight %, or from about 5 weight % to about 15 weight %, by total weight of the composition.

In some embodiments, a composition of the present invention may comprises at least one sweetener, useful for example to enhance taste of the composition. Any orally acceptable natural or artificial sweetener can be used, including without limitation dextrose, sucrose, maltose, dextrin, dried invert sugar, mannose, xylose, ribose, fructose, levulose, galactose, corn syrup (including high fructose corn syrup and corn syrup solids), partially hydrolyxed starch, hydrogenated starch hydrolysate, sorbitol, mannitol, xylitol, maltitol, isomalt, aspartame, neotame, saccharin and salts thereof (such as sodium saccharin), dipeptide-based intense sweeteners, cyclamates and the like. One or more sweeteners are optionally present in a total amount depending strongly on the particular sweetener(s) selected, but typically 0.005 weight % to 5 weight %, by total weight of the composition, optionally 0.005 weight % to 0.2 weight %, further optionally 0.05 weight % to 0.1 weight % by total weight of the composition.

In some embodiments, a composition of the present invention may comprises at least one flavorant, useful for example to enhance taste of the composition. Any orally acceptable natural or synthetic flavorant can be used, including without limitation tea flavours (such as Cool Jasmine Tea Flavour), vanillin, sage, marjoram, parsley oil, spearmint oil, cinnamon oil, oil of wintergreen (methylsalicylate), peppermint oil, clove oil, bay oil, anise oil, eucalyptus oil, citrus oils, fruit oils and essences including those derived from lemon, orange, lime, grapefruit, apricot, banana, grape, apple, strawberry, cherry, pineapple, *etc.*, bean- and nut-derived flavors such as coffee, cocoa, cola, peanut, almond, *etc*., adsorbed and encapsulated flavorants and the like. Also encompassed within flavorants herein are ingredients that provide fragrance and/or other sensory effect in the mouth, including cooling or warming effects. Such ingredients illustratively include menthol, menthyl acetate, menthyl lactate. camphor, eucalyptus oil, eucalyptol, anethole, eugenol, cassia, oxanone, α-irisone, propenyl guaiethol, thymol, linalool, benzaldehyde, cinnamaldehyde, N-ethyl-*p*-menthan-3-carboxamine, N,2,3-trimethyl-2-isopropylbutanamide, 3-(1-menthoxy)-propane-1,2-diol, cinnamaldehyde glycerol acetal (CGA), menthone glycerol acetal (MGA) and the like. One or more flavorants are optionally present in a total amount of from about 0.01 wt. % to about 5 wt. %, for example, from about 0.03 wt. % to about 2.5 weight %, optionally about 0.05 weight % to about 1.5 weight %, further optionally about 0,1 weight % to about 0.3 weight % by total weight of the composition.

A composition of the invention may comprise at least one colorant. Colorants herein include pigments, dyes, lakes and agents imparting a particular luster or reflectivity such as pearling agents. Any orally acceptable colorant can be used, including without limitation talc, mica, magnesium carbonate, calcium carbonate, magnesium silicate, magnesium aluminum silicate, silica, titanium dioxide, zinc oxide, red, yellow, brown and black iron oxides, ferric ammonium ferrocyanide, manganese violet, ultramarine, titaniated mica, bismuth oxychloride, and the like. One or more colorants are optionally present in a total amount of from about 0.001 weight % to about 20 weight %, for example, from about 0.01 weight % to about 10 wt. %, or from about 0.1 wt. % to about 5 weight %, by total weight of the composition.

The compositions of the present invention optionally comprise an antibacterial or preservative agent, such as chlorhexidine, triclosan, quatemary ammonium compounds (for example benzalkonium chloride), parabens such as methylparaben, propylparaben, or a bleaching agent such as peroxides and oxidorednctase enzymes. One or more antibacterial or preservative agent is optionally present in the composition in a total amount of from about 0.01 weight % to about 0.5 weight %, optionally about 0.05 weight % to about 0.1 weight % by total weight of the composition.

In some embodiments, the composition may comprise a fluoride ion source. Fluoride ion sources include, but are not limited to: stannous fluoride, sodium fluoride, potassium fluoride, potassium monofluorophosphate, sodium monofluorophosphate, ammonium monofluorophosphate, sodium fluorosilicate, ammonium fluorosilicate, amine fluoride such as olaflur (N'-octadecyltrimethylendimnine-N,N,N'-tris(2-ethanol)-dihydrofluoride), ammonium fluoride, and combinations thereof. In certain embodiments the fluoride ion source includes stannous fluoride, sodium fluoride, amine fluorides, sodium monofluorophosphate, as well as mixtures thereof. In certain embodiments, the oral care composition of the invention may also contain a source of fluoride ions or fluorine-providing ingredient in amounts sufficient to supply about 50 to about 5000 ppm fluoride ion, e.g., from about 100 to about 1000, from about 200 to about 500, or about 250 ppm fluoride ion. Fluoride ion sources may be added to the compositions of the invention at a level of about 0,001 wt. % to about 10 wt. %, e.g., from about 0,003 wt. % to about 5 wt. %, 0.01 wt. % to about 1 wt., or about 0.05 wt. %, However, it is to be understood that the weights of fluoride salts to provide the appropriate level of fluoride ion will obviously vary based on the weight of the counter ion in the salt, and one of skill in the art may readily determine such amounts. A preferred fluoride salt may be sodium fluoride.

The composition of the present invention optionally comprises a saliva stimulating agent useful, for example, in amelioration of dry mouth. Any orally acceptable saliva stimulating agent can be used, including without limitation food acids such as citric, lactic, malic, succinic, ascorbic, adipic, fumaric and tartaric acids, and mixtures thereof. One or more saliva stimulating agents are optionally present in saliva stimulating effective total amount.

The composition of the present invention optionally incorporates one or more further antisensitivity agents, e.g., potassium salts such as potassium nitrate, potassium bicarbonate, potassium chloride, potassium citrate, and potassium oxalate; capsaicin; eugenol; strontium salts; chloride salts and combinations thereof. Such agents may be added in effective amounts, e.g., from about 1 wt. % to about 20 wt. % by weight based on the total weight of the composition, depending on the agent chosen. The compositions of the present invention may also be used to treat hypersensitivity by blocking dentin tubules when applied to a tooth.

In some embodiments, the composition of the invention further comprises an antioxidant. Any orally acceptable antioxidant can be used, including butylated hydroxyanisole (BHA), butylated hydroxytoluene (BHT), vitamin A, carotenoids, vitamin E, flavonoids, polyphenols, ascorbic acid, herbal antioxidants, chlorophyll, melatonin, and mixtures thereof.

The composition of the present invention may additionally optionally comprise a tartar control (anticalculus) agent as provided below. Tartar control agents among those useful herein include salts of the specified agents, including alkali metal and ammonium salts. The agents include: phosphates and polyphosphates, polyaminopropanesulfonic acid (AMPS), polyolefin sulfonates, polyolefin phosphates, diphosphonates such as azacydoalkane-2,2-diphosphonates (e.g., azacycloheptane-2,2-diphosphonic acid), N-methyl azacyclopentane-2,3-diphosphonic acid, ethane-1-hydroxy-1,1-diphosphonic acid (EHDP) and ethane-1-amino-1,1-diphosphonate, phosphonoalkane carboxylic acids and. Useful inorganic phosphate and polyphosphate salts include monobasic, dibasic and tribasic sodium phosphates, sodium tripolyphosphate, tetrapolyphosphate, sodium trimetaphosphate, sodium hexaraelaphosphate and mixtures thereof. Other useful tartar control agents include polycarboxylate polymers and polyvinyl methyl ether/maleic anhydride (PVM/MA) copolymers, such as GANTREZ®.

In some embodiments, the composition of the present invention further comprises a nutrient. Suitable nutrients include vitamins, minerals, amino acids, and mixtures thereof. Vitamins include Vitamins C and D, thiamine, riboflavin, calcium pantothenate, niacin, folic acid, nicotinamide, pyridoxine, cyanocobalamin, para-aminobenzoic acid, bioflavonoids, and mixtures thereof. Nutritional supplements include amino acids (such as L-tryptophan, L-lysine, methionine, threonine, levocarnitine and L-carnitine), lipotropics (such as choline, inositol, betaine, and linoleic acid), and mixtures thereof.

The present invention also provides said oral care composition for use in a method of reducing oral malodor in an oral cavity of a subject, the method comprising applying an oral care composition of the present invention to the oral cavity.

### Examples

In the experiments described herein, volatile sulfur compounds (VSCs) are generated by incubating whole saliva (85%), deionized water (10%) and fluid thioglycollate (5%) media overnight (at least 16 hrs) at 37°C.

In order to compare the efficacy of various actives in reducing the level of VSCs, the actives under investigation are added to the saliva/water/fluid thioglicallate media mixture prior to the overnight incubation at 37°C.

In the following examples, 0.1 mol of a mouthwash formulation containing the active (or combination of actives) under investigation was mixed with 3mL of the whole saliva/water/fluid thioglycollate (FTG) media: mixture (as described above) in a container- and the mixtures allowed to incubate overnight (for at least 16 hrs) at 37°C.

The amount of volatile sulfur compounds produced by the various mixtures was determined by measuring the concentration of VSCs in the headspace above the mixture using gas chromatography and a sulfur detector. The percentage reduction in VSCs was calculated by measuring the VSCs in the headspace of a vial containing the mixture of saliva/water/FTG and the active and comparing this result to the saliva/water/FTG mixture alone (control). (The results are shown in Examples 1 to 5)

In each of the examples, below, the mouthwash formulations contained the various zinc compounds in the concentrations as shown in Table 1 Table 1A provides the composition of the placebo. Each test variant was prepared by adding the active and adjusting the water to achieve the desired concentration.

**Table 1**

| Chemical name | Zinc Oxide | Zinc Lactate | Zinc Chloride | Zinc Citrate |
|---|---|---|---|---|
| Chemical Formula | ZnO | ZnC₆H₁₀O₆ | ZnCl₂ | Zn₃(C₆H₅O₇)₂ |
| M.W. chemical (M.W.Zn = 65.38) | 81.408 | 243.53 | 136.29 | 574.37 |
| Weight % in mouthwash formulation | 0.05 | 0.14 | 0.08 | 0.1 |
| Mol. % Zn | 0.000612 | 0.000575 | 0.000587 | 0.000522 |

**Table 1A: Placebo Mouthrinse**

| **Ingredient** | **% Composition** |
|---|---|
| Demineralized water | Q.S. |
| Sorbitol | 7 |
| Glycerin | 7 |
| Propylene Glycol | 4 |
| Polyoxyl 40 Hydrogenated Castor Oil | 1 |
| Sodium Benzoate | 0,5 |
| Citric Acid Anhydrous | 0.3 |
| Flavor | 0.147 |
| Sodium Fluoride | 0.05 |
| Sweetner | 0.001 |
| Color | 0.002 |
| Total | 100 |

Where arginine (or another amino acid) was present, the concentration of the amino acid in the mouthwash formulations was 0.8 weight %.

For comparison, a placebo mouthwash formulation containing no arginine or zinc was also evaluated for efficacy in reducing VSCs. These comparative formulation was also used in an amount of 0.1 mL.

### Example 1 (Mouthwashes with and without arginine)

in this example, mouthwash formulations containing various zinc compounds, both with and without arginine, were evaluated using the experimental protocol as described above. The results are as shown below in Table 2.

**Table 2**

| **Actives present in formulation** | **% Reduction in VSCs** |
|---|---|
| Arginine (0,8 weight %) | 8 |
| Zinc oxide (0.05 weight %) | 43 |
| Zinc oxide (0.05 weight %) + arginine (0.8 weight %) | 96 |
| Zinc lactate (0.14 weight %) | 36 |
| Zinc lactate (0.14 weight %) + arginine (0.8 weight %) | 96 |
| Zinc chloride (0.08 weight %) | 42 |
| Zinc chloride (0.08 weight %) + arginine (0.8 weight %) | 97 |
| Zinc citrate (0.1 weight %) | 42 |
| Zinc citrate (0.1 weight %) + arginine (0.8 weight %) | 95 |
| Placebo | 14 |

it can be seen from these results that the addition of arginine to mouthwash formulations containing zinc ions significantly increases the VSC reducing properties of the mouthwash formulations.

There are different classes of amino acids, based on polarity and pH. For example, aspartic acid an acidic polar amino acid and glycine is a neutral non-polar amino acid. Arginine, histidine and lysine are basic polar amino acids. Histidine and lysine were tested to determine whether the effects as shown above for compositions comprising a combination of arginine and zinc ions were also shown by compositions comprising a combination of zinc ions with other basic polar amino acids (see examples 2 and 3, below). Aspartic acid and glycine were tested to determine whether or not the above effects could also be seen for a combination of zinc ions with amino acids of classes other than "basic, polar" amino acids (see examples 4 and 5, below).

### Example 2 (Mouthwashes with and without histidine)

In this example, mouthwash formulations containing various zinc compounds, both with and without histidine, were evaluated using the experimental protocol as described above. The results are as shown below in Table 3.

**Table 3**

| **Actives present in formulation** | **% Reduction in VSCs** |
|---|---|
| Histidine (0.8 weight %) | 15 |
| Zinc oxide (0.05 weight %) | 33 |
| Zinc oxide (0.05 weight %) + histidine (0,8 weight %) | 35 |
| Zinc lactate (0.14 weight %) | 35 |
| Zinc lactate (0.14 weight %) + histidine (0.8 weight %) | 32 |
| Zinc chloride (0.08 weight %) | 49 |
| Zinc chloride (0.08 weight %) + histidine (0.8 weight %) | 37 |
| Zinc citrate (0.1 weight %) | 36 |
| Zinc citrate (0.1 weight %) + histidine (0.8 weight %) | 22 |
| Placebo | 16 |

### Example 3 (Mouthwashes with and without lysine)

In this example, mouthwash formulations containing various zinc compounds, both with and without lysine, were evaluated using the experimental protocol as described above. The results are as shown below in Table 4.

**Table 4**

| **Actives present in formulation** | **% Reduction in VSCs** |
|---|---|
| Lysine (0.8 weight %) | 21 |
| Zinc oxide (0.05 weight %) | 34 |
| Zinc oxide (0.05 weight %) + lysine (0,8 weight %) | 44 |
| Zinc lactate (0.14 weight %) | 38 |
| Zinc lactate (0,14 weight %) + lysine (0.8 weight %) | 40 |
| Zinc chloride (0.08 weight %) | 43 |
| Zinc chloride (0,08 weight %) + lysine (0.8 weight %) | 43 |
| Zinc citrate (0. 1 weight %) | 38 |
| Zinc citrate (0.1 weight %) + lysine (0.8 weight %) | 43 |
| Placebo | 35 |

### Example 4 (Mouthwashes with and without aspartic acid)

In this example, mouthwash formulations containing various zinc compounds, both with and without aspartic acid, were evaluated using the experimental protocol as described above. The results are as shown below in Table 5.

**Table 5**

| **Actives present in formulation** | **% Reduction in VSCs** |
|---|---|
| Aspartic acid (0.8 weight %) | 9 |
| Zinc oxide (<i.05 weight %) | 34 |
| Zinc oxide (0.05 weight %) + aspartic acid (0,8weight %) | 31 |
| Zinc lactate (0.14 weight %) | 37 |
| Zinc lactate (0,14 weight %) + aspartic acid (0,8 weight %) | 24 |
| Zinc chloride (0.08 weight %) | 46 |
| Zinc chloride (0.08 weight %) + aspartic acid (0,8 weight %) | 31 |
| Zinc citrate (0.1 weight %) | 40 |
| Zinc citrate (0.1 weight %) + aspartic acid (0.8 weight %) | 32 |
| Placebo | 14 |

### Example 5 (Mouthwashes with and without glycine)

In this example, mouthwash formulations containing various zinc compounds, both with and without glycine, were evaluated using the experimental protocol as described above. The results are as shown below in Table 6.

**Table 6**

| **Actives present in formulation** | **% Reduction in VSCs** |
|---|---|
| Glycine (0.8 weight %) | 4 |
| Zinc oxide (0,05 weight %) | 40 |
| Zinc oxide (0.05 weight %) + glycine (0.8 weight %) | 28 |
| Zinc lactate (0.14 weight %) | 20 |
| Zinc lactate (0.14 weight %) + glycine (0.8 weight %) | 27 |
| Zinc chloride (0.08 weight %) | 33 |
| Zinc chloride (0.08 weight %) + glycine (0.8 weight %) | 34 |
| Zinc citrate (0.1 weight %) | 29 |
| Zinc citrate (0.1 weight %) + glycine (0.8 weight %) | 28 |
| Placebo | 5 |

It can be seen from Examples 2 to 5, above, that the surprisingly high efficacy of the combination of arginine and zinc ions was not shared by the combination of zinc ions with any of the amino acids histidine, lysine, aspartic acid or glycine.

## Claims

1. An oral care composition comprising:
a. arginine in free or salt form; and
b. zinc ions,
wherein the zinc ions are present in the composition at a concentration of from 0.0005 mols/100g to 0.00062 mols/100g, based on the total weight of the composition.

2. The oral care composition of claim 1, wherein the zinc ions are present in the composition at a concentration of from 0.00052 mols/100g to 0.00062 mols/100g, based on the total weight of the composition.

3. The oral care composition of claim 1 or claim 2, wherein the arginine is present in the composition at a concentration of from 0.1 weight % to 1 weight %, based on the total weight of the composition.

4. The oral care composition of claim 3, wherein the arginine is present in the composition at a concentration of from 0.5 weight % to 0.8 weight %, based on the total weight of the composition.

5. The oral care composition of any one of the preceding claims, wherein the zinc ions are provided by at least one zinc compound chosen from zinc oxide, zinc lactate, zinc chloride, zinc citrate, zinc acetate, zinc borate, zinc butyrate, zinc carbonate, zinc formate, zinc gluconate, zinc glycerate, zinc glycolate, zinc phosphate, zinc picolinate, zinc proprionate, zinc salicylate, zinc silicate, zinc stearate, zinc tartrate, zinc undecylenate, zinc phosphate, zinc ricinoleate, zinc nitrate, and zinc sulfate.

6. The oral care composition of claim 5, wherein the zinc ions are provided by a zinc compound selected from zinc oxide, zinc lactate, zinc chloride and zinc citrate.

7. The oral care composition of any one of the preceding claims, wherein the arginine is present as free arginine.

8. The oral care composition of any one of claims 1 to 6, wherein the arginine is present as an arginine salt selected from arginine bicarbonate, arginine hydroxide, arginine carbonate, arginine phosphate and mixtures thereof.

9. The oral care composition of any preceding claim, wherein the composition further comprises an orally acceptable carrier.

10. The oral care composition of any one of the preceding claims, wherein the composition is a toothpaste, a gel, a mouthwash, a mouthrinse, a lozenge, a spray, a gum, a tablet, or a film.

11. The oral care composition of claim 10, wherein the composition is a mouthwash.

12. The oral care composition of claim 10, wherein the composition is a toothpaste.

13. The oral care composition of any preceding claim, for use in reducing oral malodor in an oral cavity of a subject, wherein the oral care composition is to be applied to the oral cavity.

## Patentansprüche

1. Mundpflegezusammensetzung, umfassend:
a. Arginin in freier oder Salzform; und
b. Zinkionen,
wobei die Zinkionen in der Zusammensetzung in einer Konzentration von 0,0005 Mol/100g bis 0,00062 Mol/100g basierend auf dem Gesamtgewicht der Zusammensetzung vorliegen.

2. Mundpflegezusammensetzung nach Anspruch 1, wobei die Zinkionen in der Zusammensetzung in einer Konzentration von 0,00052 Mol/100g bis 0,00062 Mol/100g basierend auf dem Gesamtgewicht der Zusammensetzung vorliegen.

3. Mundpflegezusammensetzung nach Anspruch 1 oder Anspruch 2, wobei das Arginin in der Zusammensetzung in einer Konzentration 0,1 Gew.-% bis 1 Gew.-% basierend auf dem Gesamtgewicht der Zusammensetzung vorliegt.

4. Mundpflegezusammensetzung nach Anspruch 3, wobei das Arginin in der Zusammensetzung in einer Konzentration von 0,5 Gew.-% bis 0,8 Gew.-% basierend auf dem Gesamtgewicht der Zusammensetzung vorliegt.

5. Mundpflegezusammensetzung nach einem beliebigen der voranstehenden Ansprüche, wobei die Zinkionen durch mindestens eine aus Zinkoxid, Zinklactat, Zinkchlorid, Zinkcitrat, Zinkacetat, Zinkborat, Zinkbutyrat, Zinkcarbonat, Zinkformiat, Zinkgluconat, Zinkglycerat, Zinkglycolat, Zinkphosphat, Zinkpicolinat, Zinkproprionat, Zinksalicylat, Zinksilicat, Zinkstearat, Zinktartrat, Zinkundecylenat, Zinkphosphat, Zinkricinoleat, Zinknitrat und Zinksulfat ausgewählte Zinkverbindung bereitgestellt werden.

6. Mundpflegezusammensetzung nach Anspruch 5, wobei die Zinkionen durch eine aus Zinkoxid, Zinklactat, Zinkchlorid und Zinkcitrat ausgewählte Zinkverbindung bereitgestellt werden.

7. Mundpflegezusammensetzung nach einem beliebigen der voranstehenden Ansprüche, wobei das Arginin als freies Arginin vorliegt.

8. Mundpflegezusammensetzung nach einem beliebigen der Ansprüche 1 bis 6, wobei das Arginin als ein aus Argininbicarbonat, Argininhydroxid, Arginincarbonat, Argininphosphat und Gemischen davon ausgewähltes Argininsalz vorliegt.

9. Mundpflegezusammensetzung nach einem beliebigen voranstehenden Anspruch, wobei die Zusammensetzung des Weiteren einen oral verträglichen Träger umfasst.

10. Mundpflegezusammensetzung nach einem beliebigen der voranstehenden Ansprüche, wobei die Zusammensetzung eine Zahnpasta, ein Gel, ein Mundwasser, eine Mundspülung, eine Lutschtablette, ein Spray, ein Gummi, eine Tablette oder ein Film ist.

11. Mundpflegezusammensetzung nach Anspruch 10, wobei die Zusammensetzung ein Mundwasser ist.

12. Mundpflegezusammensetzung nach Anspruch 10, wobei die Zusammensetzung eine Zahnpasta ist.

13. Mundpflegezusammensetzung nach einem beliebigen voranstehenden Anspruch, zur Verwendung beim Verringern von Mundgeruch in einer Mundhöhle eines Subjektes, wobei die Mundpflegezusammensetzung auf die Mundhöhle anzuwenden ist.

## Revendications

1. Composition de soin buccal comprenant :
a. de l'arginine sous forme libre ou sous forme de sel ; et
b. des ions zinc,
dans laquelle les ions zinc sont présents dans la composition à une concentration de 0,0005 mole/100 g à 0,00062 mole/100 g, par rapport au poids total de la composition.

2. Composition de soin buccal selon la revendication 1, dans laquelle les ions zinc sont présents dans la composition à une concentration de 0,00052 mole/100 g à 0,00062 mole/100 g, par rapport au poids total de la composition.

3. Composition de soin buccal selon la revendication 1 ou la revendication 2, dans laquelle l'arginine est présente dans la composition à une concentration allant de 0,1 % en poids à 1 % en poids, par rapport au poids total de la composition.

4. Composition de soin buccal selon la revendication 3, dans laquelle l'arginine est présente dans la composition à une concentration allant de 0,5 % en poids à 0,8 % en poids, par rapport au poids total de la composition.

5. Composition de soin buccal selon l'une quelconque des revendications précédentes, dans laquelle les ions zinc sont fournis par au moins un composé de zinc choisi parmi l'oxyde de zinc, le lactate de zinc, le chlorure de zinc, le citrate de zinc, l'acétate de zinc, le borate de zinc, le butyrate de zinc, le carbonate de zinc, le formiate de zinc, le gluconate de zinc, le glycérate de zinc, le glycolate de zinc, le phosphate de zinc, le picolinate de zinc, le propionate de zinc, le salicylate de zinc, le silicate de zinc, le stéarate de zinc, le tartrate de zinc, l'undécylénate de zinc, le phosphate de zinc, le ricinoléate de zinc, le nitrate de zinc et le sulfate de zinc.

6. Composition de soin buccal selon la revendication 5, dans laquelle les ions zinc sont fournis par un composé de zinc choisi parmi l'oxyde de zinc, le lactate de zinc, le chlorure de zinc et le citrate de zinc.

7. Composition de soin buccal selon l'une quelconque des revendications précédentes, dans laquelle l'arginine est présente sous forme d'arginine libre.

8. Composition de soin buccal selon l'une quelconque des revendications 1 à 6, dans laquelle l'arginine est présente sous la forme d'un sel d'arginine choisi parmi le bicarbonate d'arginine, l'hydroxyde d'arginine, le carbonate d'arginine, le phosphate d'arginine et leurs mélanges.

9. Composition de soin buccal selon l'une quelconque des revendications précédentes, dans laquelle la composition comprend en outre un support acceptable par voie orale.

10. Composition de soin buccal selon l'une quelconque des revendications précédentes, dans laquelle la composition est un dentifrice, un gel, un bain de bouche, un rince-bouche, une pastille, un aérosol, une gomme, un comprimé ou un film.

11. Composition de soin buccal selon la revendication 10, dans laquelle la composition est un bain de bouche.

12. Composition de soin buccal selon la revendication 10, dans laquelle la composition est un dentifrice.

13. Composition de soin buccal selon l'une quelconque des revendications précédentes, destinée à être utilisée pour réduire la mauvaise haleine dans la cavité buccale d'un sujet, la composition de soin buccal devant être appliquée à la cavité buccale.
